# EUROPEAN PATENT SPECIFICATION

(11) **EP 0 594 581 B1**
(45) Date of publication and mention of the grant of the patent: **01.07.1998**
(21) Application number: 91902953.8
(22) Date of filing: 22.01.1991
(51) Int. Cl.: C07J 17/00, A61K 31/58

(54) **PRODUCTION OF GLYCOSIDES, ESPECIALLY OF STEROIDAL GLYCOSIDES**
VERFAHREN ZUR HERSTELLUNG VON GLYKOSIDEN, INSBESONDERE STEROIDGLYKOSIDEN
PRODUCTION DE GLYCOSIDES, EN PARTICULIER DE GLYCOSIDES STEROIDIENS

(30) Priority: 23.01.1990 DE 4001895
(43) Date of publication of application: 04.05.1994
(73) Proprietor: Klemke, Rudolf Erich, Dipl.-Chem., D-7763 Öhningen (DE)
(72) Inventor: KLEMKE, Rudolf, Erich, D-7709 Hilzingen-Riedheim (DE)
(74) Representative: Viering, Jentschura & Partner
(86) International application number: PCT/EP91/00115
(87) International publication number: WO 91/11452

(56) References cited:
- DE-A- 3 127 933
- FR-A- 2 007 410
- Liebigs Annalen der Chemie 1985, VCH Verlagsgesellschaft mbH, (Weinheim, DE), J. Thiem et al.: "Untersuchungen zur Darstellung von Desoxyzucker-Steroid-glycosiden", pages 2135-2150
- Carbohydrate Research, vol. 29, no. 2, August 1973, (Amsterdam, NL), S. Honda et al.: "Preparation of O-(2-deoxy-x-D-arabino-hexopyranosyl)-(1 -6)-D-glucose by the oxiodination-hydrogenation method", pages 488-491
- Carbohydrate Research, vol. 92, 1981, Elsevier Scientific Publishing Co., (Amsterdam, NL), P.J. Garegg et al.: "Novel glycosylation reagents: synthesis of disaccharides containing 2-deoxy-2-iodo-x-D-talopyranosyl groups", pages 157-159

## Description

### Field of the invention

This invention relates to a method for the production of hex-2-ene-pyranosides of alcohols and phenols by glycosylation of an alcohol and/or a phenol, preferably a hydroxy-steroid, as well as a steroidal hex-2-ene-pyranoside and a medicament containing the same.

An object of the invention is a method for the production of hex-2-ene-pyranosides of alcohols and phenols and the steroidal hex-2-ene-pyranoside according to the invention as well as the provision thereof as a therapeutically-active substance, medicaments based on this steroidal hex-2-ene-pyranoside for control or prevention of diseases, especially for the manufacture of a medicament for the treatment of cancer diseases, geriatric diseases, states of hyperactivity and/or states of diminished activity.

### Background of the invention

The glycosylation of alcohols and/or phenols and particularly that of hydroxy-steroids is known per se; however, often there arise undesired ortho esters as e.g. described in chemical Abstracts, Vol. 105, 1986, 172882s. A method which allows the content of this undesired ortho ester to be decreased is disclosed in Chemical Abstracts, Vol. 104, 1986, 22511g (Liebigs Ann. Chem. 1986, 717-730); however, this method does not allow the complete avoidance of the formation of ortho esters, and, further, a pivaloylglucopyranosylbromide must be used, wherein the pivaloyl groups function as protecting groups to suppress the formation of ortho esters. The reaction of the glycoside with the steroid proceeds by means of silver oxide or silver carbonate catalysts.

A method for coupling 3,4-di-O-acetyl-rhamnal to cholesterol using BF₃ as a catalyst has been described by Thiem et al., Liebigs Ann. Chem. 1985, 2135-2150.

Further, FR-A-2.007.410 discloses a method for coupling 1,2-glucals to steroidal compounds under acidic conditions.

The use of α-halogen-tetraacetylglucose which is commonly used for the glycosylation of steroids, especially that of cholesterol, necessitates the use of expensive and/or toxic reaction catalysts, such as Ag₂O, Ag₂CO₃, PbCO₃, Hg(CN)₂ etc., which frequently prohibits its technical application on a larger scale. Furthermore, these glycosylation procedures generally constitute multistage processes which also lead to the production of α- as well as to β-glycosylation.

This invention solves the problem of providing novel hex-2-ene-pyranosides of alcohols and phenols, especially a steroidal hex-2-ene-pyranoside for pharmacological application. The glycosylation for the production thereof proceeds in one step and without extensive laboratory measures, such as nitrogen gassing and/or low temperatures, and avoids halogenated glycosides and the utilization of toxic reaction catalysts, such as for example Ag₂O, Ag₂CO₃, PbCO₃, Hg(CN)₂ etc. and avoids the formation of ortho esters.

### Summary of the invention

It has been surprisingly found that members selected from the group consisting of alcohols and phenols and preferably hydroxy-steroids, wherein hydroxy-steroids are to be understood as members selected from the group consisting-of steroidal alcohols and steroidal phenols, can be reacted with a glucal derivative in the presence of molecular iodine as a catalyst in one step to yield a hex-2-ene-pyranoside in high yield. Thus there is no need for expensive and toxic reagents in this reaction step. Furthermore, a steroidal hex-2-ene-pyranoside has been found which is obtainable by this method and which can be employed as a highly efficient medicament, especially as an anti cancer agent, in geriatric medicine, as a sedative and/or activity-enhancing agent, as well as the use of said hex-2-ene-pyranoside for the manufacture of a pharmaceutical compound treating symptoms of at least one member selected from the group consisting of cancer disease, geriatric disease, states of restlessness and states of weakness, the compound having the formula :

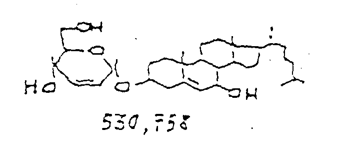

In the accompanying drawings with reference to preferred examples of the invention:
Diagram 1 is an infrared spectrum of the glucal used in the reaction of Example 1;
Diagram 2 is an infrared spectrum of the glycosylation product of Example 1;
Diagram 3 is an NMR-sectrum of the same glycosylation product of example 1;
Diagram 4 and 5 are the IR-spectrum and the NMR-spectrum, respectively of the ketone product of example 2;
Diagrams 6 and 7 are the IR-spectrum and the NMR-spectrum, respectively, of the 7β-OH Cholesterol product of example 3; and

Diagram 8 is a plot showing the tumor cell groth inhibition by selected concentrations of 7β-OH cholesterol in cell culture field.

The K-562 tumor cell growth inhibition by Tumosterone = 7-β-OH-Cholesterol is shown wherein the points represent average values from 3 single measured values. The y-axis represents the extinction as dimension figure for the number of cells. The x-axis represents the dilution, wherein:

| | |
|---|---|
| for 1/10 | 0,4mg TU/ml culture fluid |
| | 0,9% ethanol present in culture |
| for 1/80 | 0,03mg TU/ml culture fluid : |
| | 0,10% ethanol present in culture |
| for 1/320 | 0,007mg TU/ml culture fluid |
| | 0,030% ethanol present in culture |

The initial check and blank check are carried out as a incubation for 72 hrs at 37°C.

### Detailed description of the invention

According to one preferred embodiment of the method of the invention, an oxysteryl compound, preferably a 3β-ol sterol compound, more preferably a delta⁵-3β-ol steroid compound such as a cholesterol, (e.g., delta⁵-cholesten-3β-ol) is glycosylated by reaction with 3,4,6-tri-O-acetyl-D-glucal in an inert solvent in the presence of molecular iodine as a catalyst. The reaction is achieved in one single step and in high yield. Thus a double bond which is strongly hindered by the C₄, C₆-acetyl groups and thus being inert, is introduced between C₂=C₃ of the glycosidic part of the molecule, whereby the delta⁵ double bond of the perhydro-cyclopentanophenanthrene skeleton remains unchanged. Furthermore, the reaction of the unsaturated glycoside which is obtained as an intermediate to functional cholesterol derivatives is performed according to the method of this invention. Functional groups can be introduced into the perhydro-cyclopentano-phenanthrene skeleton of said unsaturated acetoglycoside, wherein the α-bond of the acetoglycoside at the same time functions as a protecting group for the original OH-group at C₃ of the phenanthrene skeleton.

In contrast to the analytical procedure for the iodometric assay of vinyl ethers by ionized iodine in alcohol with formation of the corresponding iodoacetals according to S. Siggia and R. L. Edsberg, Ind. Eng. Chem. Anal. 20, 762 (1948), thereby using ionized iodine in the reaction, the method according to this invention makes use of iodine being molecularly dissolved in inert solvents such as for example CH₂Cl₂ dichloromethane, CHCl₃ chloroform, CCl₄ carbon tetrachloride, C₆H₄(CH₃)₂ xylene, C₆H₃(CH₃)₃ mesitylene, C₆H₅CH(CH₃)₂ cymene, C₆H₁₂ cyclohexane and methyl derivatives thereof, as well as ligroin, petroleum ether and saturated hydrocarbons, such as for example n-pentane or n-heptane, preferably C₆H₆ benzene or C₆H₅CH₃ toluene.

The method according to the invention is applicable to the glycosylation of hydroxy compounds in general and broadly, e.g. all compounds with free alkoholic HO-groups as for example prim., sec., or tert. alcoholic groups, aliphatic, aliphatic-aromatic or aromatic. Preferred hydroxy compounds for glycosylation comprise cholesterols, bile salts, steroid hormones, and vitamin D compounds and precursors as described in Stryer's Biochemistry, 3rd Ed. pp. 559-570, Freeman and Company, New York, 1988, incorporated herewith by reference.

Specifically steroid derivative such as: Cholic acid and derivatives, 25-hydroxy-cholesterol, 25-hydroxy-calciferol, Pregnenolone, 17α-hydroxy-pregnenolone, 17α-hydroxyprogesterone, 11-desoxy-corticosteron, 11-desoxy-cortisol, corticosterone, cortisol, cortisone, androsterone, testosterone, estrone, 17β-estradiol, estratriol-3, 16α, 17β, 3α, 5β-tetrahydro-corticosterone, urocortisol and allocortolone preferably cyclopentano-perhydrophenanthrene compounds having the delta⁵-3β-OH steryl moity.

The method according to this invention is preferably directed to the reaction of 3,4,6-tri-O-acetyl-D-glucal with delta⁵-cholesten-3β-ol with a catalytic amount of molecularly dissolved iodine in one of the aforementioned solvents, thereby introducing a double bond between C-atoms 2 and 3 while eliminating the acetyl group sited at C₃, instead of introducing an iodine atom at C₂ in the glycosidic part of the resulting cholesterylhex-2-ene-pyraonoside. The iodine being utilized in a catalytic amount is quantitatively titrated back by, e.g., 0.1 N aqueous sodium thiosulphate (Na₂S₂O₃). This reaction is followed by IR-spectroscopy, and is complete only when the peak of the glucal at 1650 cm⁻¹ has disappeared.

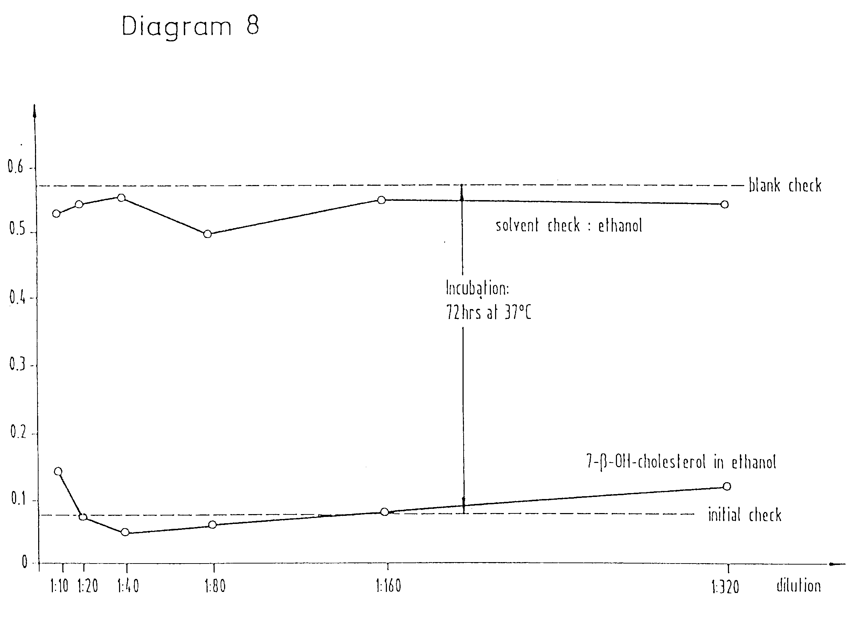

In a further step, the product obtained by the foregoing reaction can be converted by oxidation of the steroidal part into the α-glycosylated 7-keto-cholesterol. The oxidation is accomplished with an oxidizing agent, which preferably contains chromium, with pyridine-chromium trioxide (C₅H₅N)₂CrO₃ or pyridine-chlorochromate (C₅H₅NHCrO₃)Cl being preferred and t-butyl chromate being especially preferred. The inert glycosidic double bond between C₂=C₃ thereby remains intact as it is shielded by the C₆, C₄ acetyl groups The reduction of this 7-ketone with suitable, preferably complex metal hydrides, such as e.g. NaBH₄, LiBH₄, KBH₄ and preferably LiAlH₄, leads, after e.g. chromatographic separation of the C₇ α-hydroxy derivative, the 3β-O-(4,6-dihydroxy-2,3-dideoxy-D-erythro-α-hex-2-ene-pyranosyl)-delta⁵-cholesten-7α-ol, with a suitable solvent mixture, preferably a mixture consisting of dichloromethane 1 : acetone 1, to the steroidal glycoside according to this invention, to the 3β-O-(4,6-dihydroxy-2,3-dideoxy-D-erythro-α-hex-2-ene-pyranosyl)-delta⁵-cholesten-7β-ol of formula 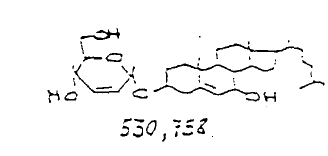 This compound posesses valuable pharmacological properties, in particular it exhibits a cell-destructive activity - free of side effects - on malignant cells and a drive-enhancing activity as well as a tranquilizing activity. The steroidal component, the delta⁵-cholesten-3β,7β-diol, constitutes an own steroid of the thymus gland being a native signal substance of the cellulary immune response which previously has been successfully employed in the treatment (free of side effects) of cancer diseases of all phenotypes. Whereas the delta⁵-cholesten-3β,7α-diol is formed in the liver as the first degradation product of cholesterol and posesses no physiologi-cal activity; the delta⁵-cholesten-3β,7β-diol is formed in the thymus gland of all mammals as a universal signal substance of their own immune defence. It owes its activity, which is solely directed to malignant cell surfaces, to the fact that it is bound unspecifically by LDL (low density lipoproteins), which are responsible for the essential transport of cholesterol into the interior of the cell and for the construction of the cell membranes, and that it is transferred by the latter ones, presumably via the NK-cells (natural killer cells) onto the cell membranes of deviated tissue, particularly onto cancerous tissue. As, in contrast to normal soma cells, the receptors of LDL on the surface of cancer cells are degeneratively modified, having undergone a modification of their spatial structure, the 7β-hydroxycholesterol effects a blocking of the receptors modified in this way. This is comparable to the plug of a bottle, wherein the cancer cell is cut of from the supply of the vital cholesterol. Hence it follows that an osmotic excess pressure builds up in the interior of the cancer cell, finally leading to the colloid-osmotic induced rupture of the cancer cell. The cytoplasma of the cancer cell is then forced out. Thus the cancer cell ceases to exist (Diagram. 8).

This method, lasting only for about 8 to 10 minutes, has been investigated microscopically and recorded by Alex Matter (Microcinematographic and electron microscopic analysis of target cell lysis induced by cytotoxic T lymphocytes, Immunology 36, 179 - 190 (1979)). No statement concerning the chemical nature of the body's own active substance is made.

7β-Hydroxy-cholesterol was detected, together with progesterone, 11β-hydroxy-progesterone, cortexone and 7-keto-cholesterol, in thymus extracts for the first time in 1976 by Klemke (unpublished results), using the antimony trichloride reaction for stenols, IR-spectroscopy and NMR-spectroscopy. Lateron Reisch and El Shakary, Scientia Pharmaceutica 50, 75-78 (1982) confirmed these findings after the group of J. P. Beck in Strasbourg, J. Chem. Res. (S) 1977, 217 - 219, had previously found that 7β-hydroxy-cholesterol constitutes the antiproliferatory active substance of a very ancient Chinese drug, the Bombyx cum Botryte, a silkworm (Bombyx mori) having been killed by a microscopic fungus (Botrytis bassiana Balls). Further details have been published in Vol. 32/ TUMOSTERON "Schriftenreihe Krebsgeschehen" of the Verlag für Medizin, Heidelberg 1986. The delta⁵-cholesten-3β,7β-diol was recognized as a biochemical signal compound of the body's own immune defence system. In contrast to the conventional cytotoxic treatment of cancer diseases this turns out to be completely non-toxic and to be capable to eliminating cancer cells of any phenotype and not affecting healthy cells.

It is true that a glycosylated cholesterol is known from Chemical Abstracts Vol. 97, 1982 6734s, which possibly might constitute a neoplastic inhibitor; however this molecule has in its glycosidic moiety at C₂ a bulky 2-chloroethyl-aminocarboxamido group and at C₇ of the cholesterol the 7β-hydroxy group is lacking. This latter group, however, is important for the activity of the steroidal glycoside according to the invention, as this steric array is also important for the respective cellular receptor.

The compound obtainable by a method according to the invention can be used as a medicament in the form of pharmaceutical preparations comprising this compound in admixture with a pharmaceutically acceptable carrier. One skilled in the art of preparing formulations can readily select the proper form and method of administration depending upon the particular characteristics of the compound selected, the disease state to be treated, the stage of disease, and other relevant circumstances. These preparations can be administrated orally, e.g. in the form of tablets, dragees, gelatin capsules, soft capsules, solutions, emulsions or suspensions or parenterally, e.g. in the form of injectable solutions or topically, e.g. in the form of cream. The compound can be administered alone or in the form of a pharmaceutical composition in combination with pharmaceutically acceptabel carriers, preservatives, solubilizers, stabilizers, humectants, emulsifiers, sweetening agents, dyes, scents, salts to modify the osmotic pressure, buffers, coating agents, antioxidants such as for example tocoquinones (tocopheroles), glutathione, cystein, ascorbic acid sodium salt etc.

The carriers mentioned above may constitute pharmaceutically inert anorganic or organic materials. Examples of carriers for tablets, capsules and hard gelatine capsules include lactose, maize-starch or derivatives thereof, talcum, stearic acid or salts thereof. Examples of carriers for soft gelatine capsules are vegetable oils, waxes, fats, semi-solid and liquid polyols. Examples of carriers for the manufacture of solutions or syrups include water, polyols, saccharose, inverted sugar and glucose. Examples of carriers for injectable solutions include water, alcohols, polyols, glycerol and vegetable oils. The pharmaceutical preparations may also comprise conventional pharmaceutical adjuvants such as preservatives, solubilizers, stabilizers, humectants, emulsifiers, sweetening agents, dyes or scents, salts to modify the osmotic pressure, buffers, coating agents or antioxidants. They may also include other therapeutically valuable ingredients.

The pharmaceutical preparations may be manufactured by admixing the compound according to this invention, if desired in combination with other therapeutically valuable substances, with an acceptable pharmaceutical carrier and, if desired, with a pharmaceutical adjuvant, and transforming the admixture into the desired form for administration.

### Dosages:

In the treatment of cancer a dosage of at most 80 mg per day, preferably 10 mg to 30 mg per day, more preferable 10 mg to 20 mg.

For the purpose of parenteral therapeutic administration, the compound of the present invention may be incorporated into a solution or suspension. These preparations should contain at least 0.1 % of a compound of the invention, but may varied to be between 0.1 % and about 50 % of the weight thereof. The amount of this inventive compound presents in such compositions is such that suitable dosage will be obtained. Preferred compositions and preparations according to the present invention are prepared so that a parenteral dosage unit contains between 5 mg to 80 mg, more preferred 5 to 40 mg, most preferred 10 to 40 mg.

A therapeutically effective dose can be readily determined by the attending diagnosticians, as one skilled in the art, by the use of conventional techniques and by observing results obtained under analoguous circumstances. In determing the therapeutically effective dose, a number of factors are considered by the attending diagnostician, including, but not limited to: the species of mammals, the size, age and general response of the individual patient; the particular compound administered; the mode of administration; the biovailability characteristics of the preparation administered; the dose regimen selected; the use of concomitant medication; and other relevant circumstances. A therapeutically effective amount of a compound according to the invention is expected to vary from about 0.07 mg per 1 kg of body weight per day (mg/kg/day) to about 1.25 mg/kg/day. Preferred amounts are expected to vary from about 0.15 mg/kg/day to about 0.3 mg/kg/day.

The reaction steps described subsequently are disposed as follows:

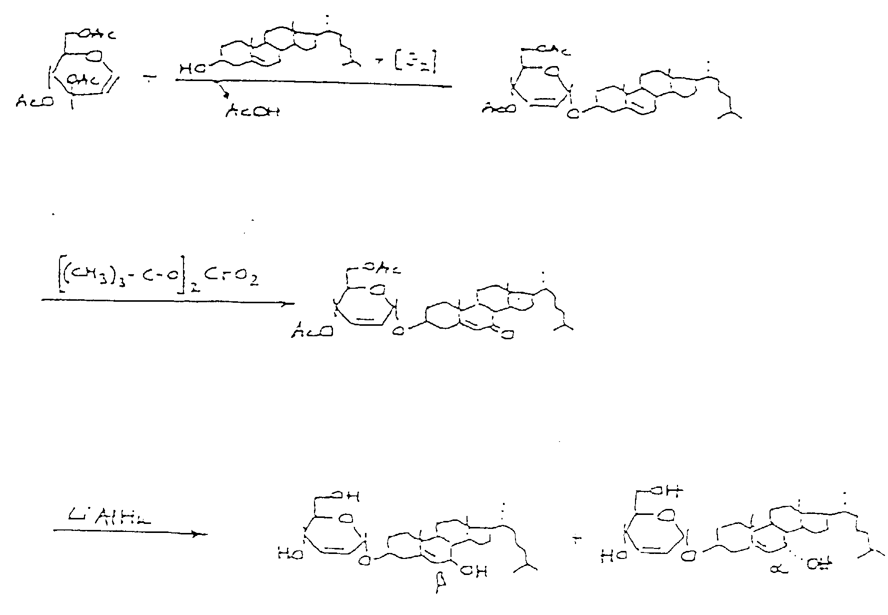

### Example 1

### Preparation of 3β-O-(4,6-O-acetyl-2,3-dideoxy-D-erythro-α-hex-2-ene-pyranosyl)-delta⁵-cholestene

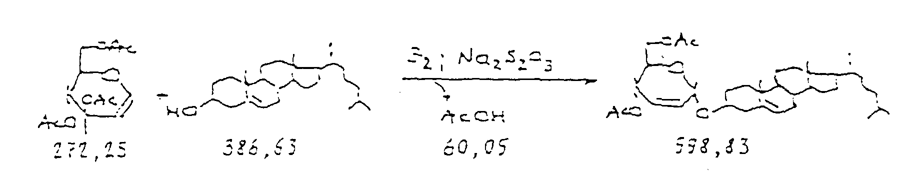

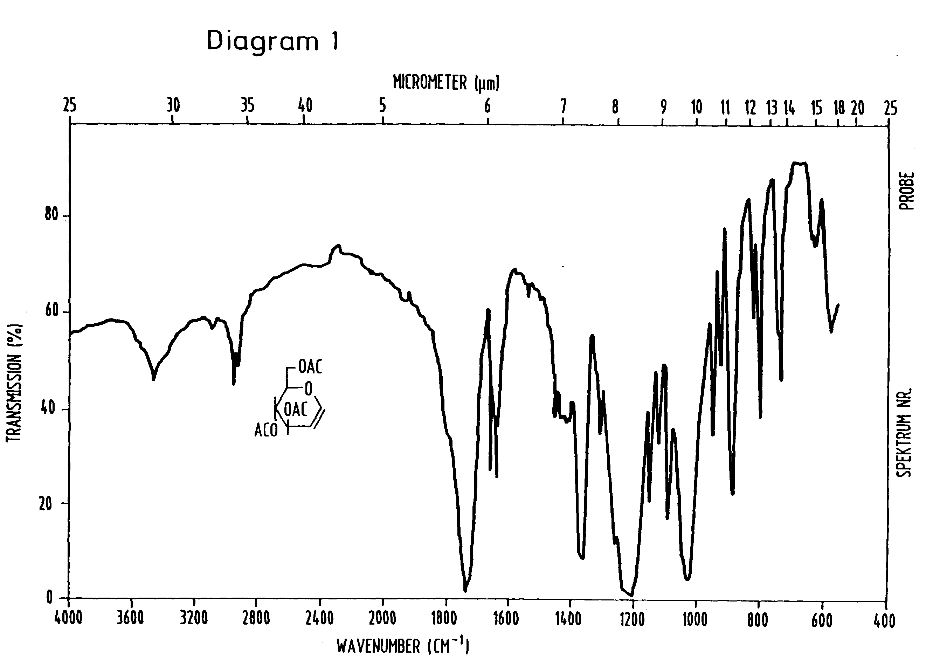

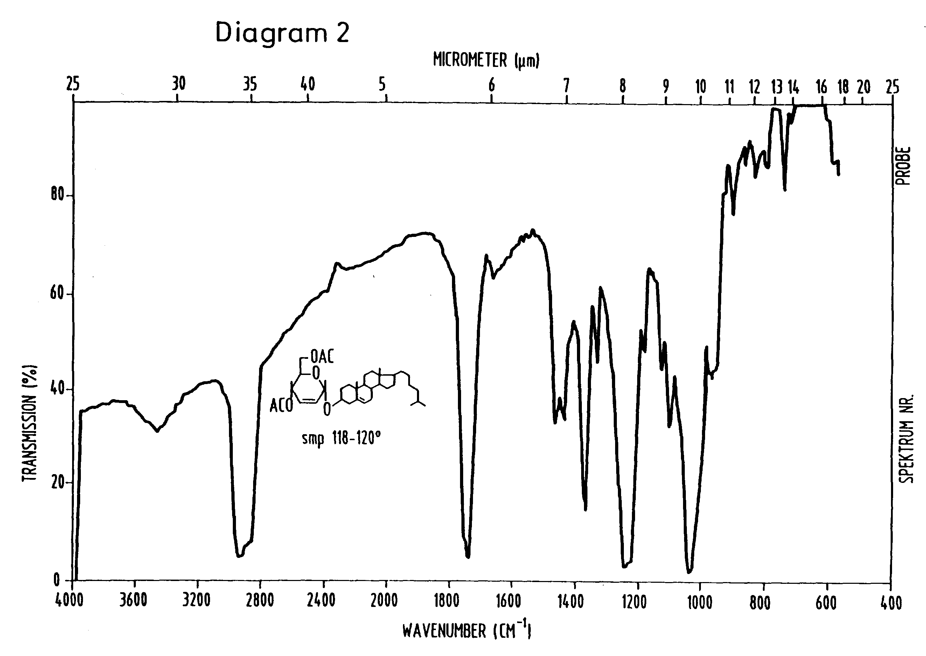

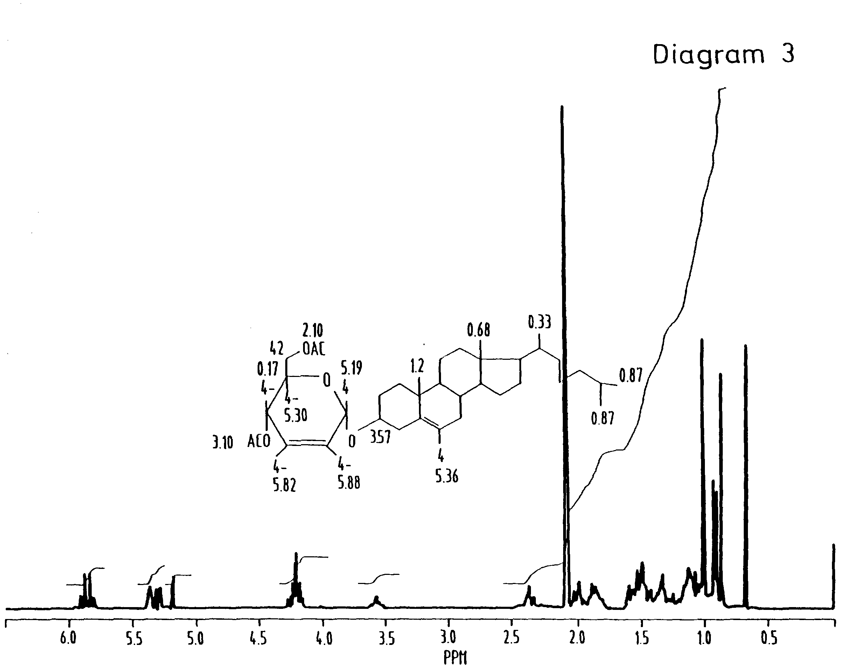

5.0 g (= 0.02 mole) molecular iodine were dissolved with stirring in 300 ml benzene in a 2-litre three-necked flask fitted with stirrer, reflux condenser and thermometer. To the wine-red solution thus obtained was added the solution of 27.2 g (= 0.10 mole) 3,4,6-tri-O-acetyl-D-glucal and 38.6 g (= 0.10 mole) delta⁵-cholesten-3β-ol in 700 ml of benzene. In the course of 2 hours the mixture was heated to 70-75 °C. The reaction was monitored by IR-spectroscopy; it was terminated only when the peak of the glucal at 1650 cm⁻¹ (Diagram 1) has disappeared. The red colour of the reaction solution is not significant. After removal of the flask heater the reaction solution is rapidly cooled in a water-bath to about 20-30 °C. After transfer into a 2-litre separatory funnel the cooled wine-red reaction solution was extracted until complete discoloration with 500 ml + 10% of 0.1 N = 12.5 g + 10 % = 13.8 g aqueous solution of Na₂SO₄, washed twice with water, treated with activated carbon, dried over anhydrous Na₂SO₄ and the solvent is distilled off, finally in vacuo.
Crude yield: 58.3 g (= 97.4% th.).
The raw product is recrystallized from 2 litres of CH₃OH. Yield: 56.95 g (= 95.1% th.)
Mp: 118-120 °C
IR-spectrum: Diagram 2
NMR-spectrum: Diagram 3

### Example 2

### Preparation of 3β-O-(4,6-O-acetyl-2,3-dideoxy-D-erythro-α-hex-2-ene-pyranosyl)-delta⁵-cholesten-7-one

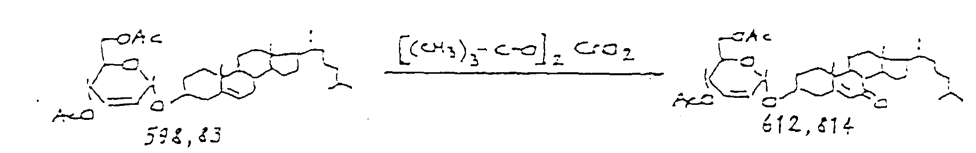

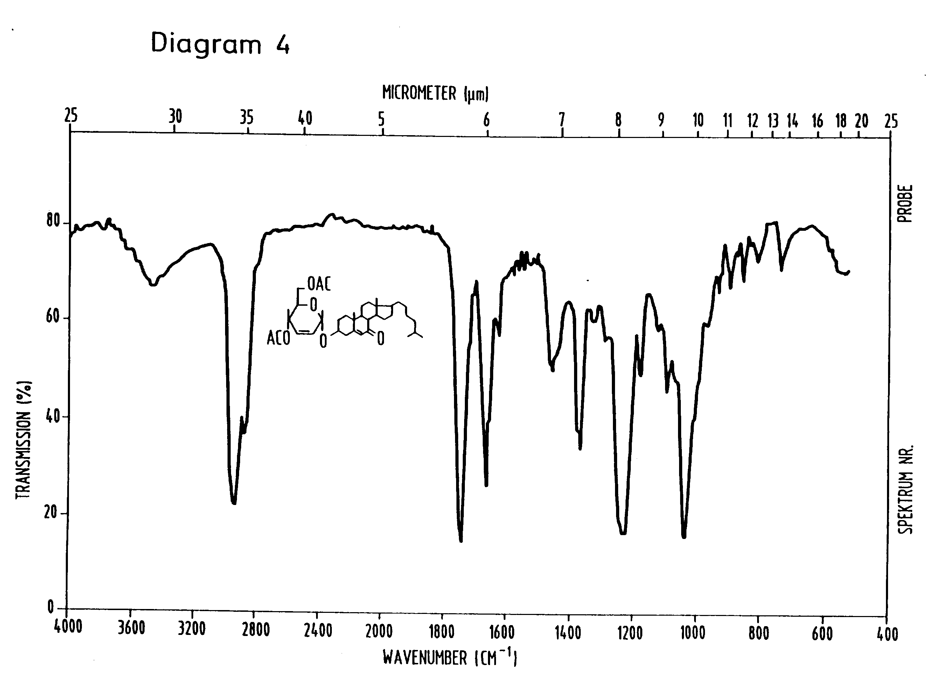

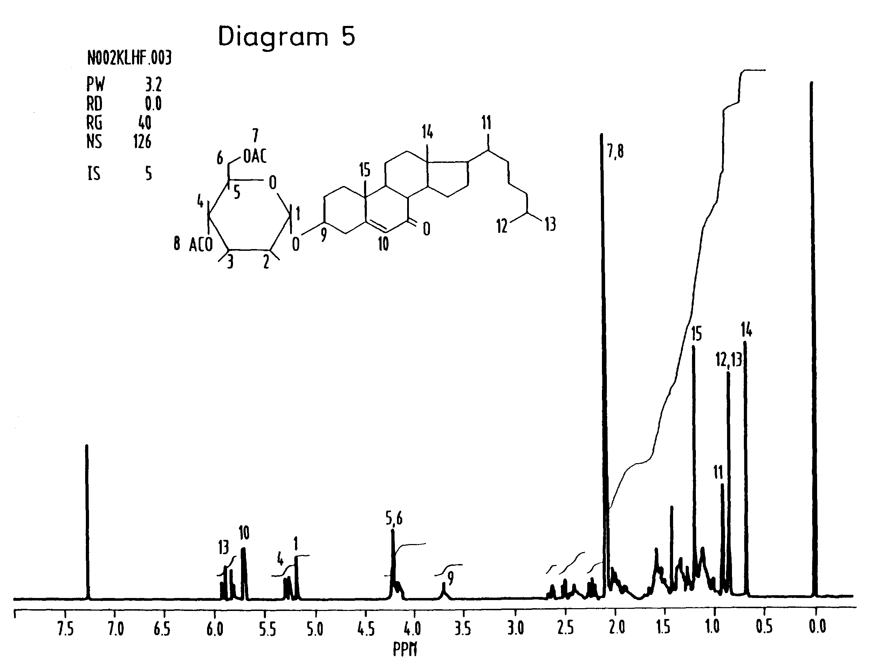

In a 250 ml three-necked flask fitted with reflux condensor, dropping funnel, thermometer arid magnetic stirrer 6.00 g (= 0.01 mole) of the unsaturated glycoside from Example 1 of mp 118-120 °C were dissolved in 45 ml of CCl₄ and heated to boiling (80 °C). In the course of 30 minutes the mixture of 10 ml Ac₂O (acetic anhydride) and 40 ml t-butyl chromate, prepared according to the Annex, was slowly added dropwise to the boiling solution and stirred for another 10 hours at the boiling point. After cooling, a solution of 6.0 g oxalic acid in 60 ml water was added dropwise in the course of 45 minutes at 5 °C to 10 °C in an ice-bath followed by 4.2 g solid oxalic acid. Stirring was then continued for another 2 hours. Thereafter separation took place in the separating funnel, the upper dark aqueous phase being extracted twice with CCl₄, the combined CCl₄-solutions extracted with water, saturated solution of NaHCO₃ and then with water again, in this order, and dried over Na₂SO₄. Finally the solution was discolored with activated carbon. After concentration in vacuo the straw-yellow residue was dissolved in 25 ml of a mixture consisting of cyclohexane 40 : ethyl acetate 10 : chloroform 1 and chromatographed on a silica gel column (diameter 2.5 cm; height 25 cm), charged with 60 g of silica gel 40 (Merck Article 10180) and the same solvent mixture.
Yield: Fraction 1: 1.8 g (= 30.1 % of theory) unchanged starting material.
Fraction 2: 4.2 g (= 68.5% of theory) 7-keto-compound
Mp: 113-115 °C
IR-spectrum: Diagram 4
NMR-spectrum: Diagram 5

### Annex:

### Preparation of t-butyl chromate

In a 500 ml beaker, 187.2 g (= 2.5 mole) t-butanol of mp 24.5 °C were warmed to 28 °C and melted. To this melt, 74 g (= 0.74 mole) of CrO₃ were added by using a thermometer as a stirring bar. In order to keep the reaction temperature below 30 °C, occasional cooling with ice-water was necessary. The liquid reaction product was- diluted in a separating funnel with 520 ml of CCl₄ and left to stand overnight. This standing is important to allow clarification of the solution. The following morning, the upper dark layer was separated. The clear CCl₄-solution was dried with 50 g of anhydrous Na₂SO₄, filtered and the Na₂SO₄ washed with 320 ml of CCl₄. Thereafter, the combined CCl₄-solutions were ccncentrated to 400 ml in vacuo in a water-bath at a temperature of 40 °C to 45 °C, wherein excess t-butanol and CCl₄ were both distilled azeotropically. The solution thus obtained may be kept unchanged in the refrigerator at -1 °C for at least one month.

### Example 3

### Preparation of 3β-O-(4,6-Hydroxy-2,3-dideoxy-D-erythro-α-hex-2-ene-pyranosyl)-delta⁵-cholesten-7β-ol

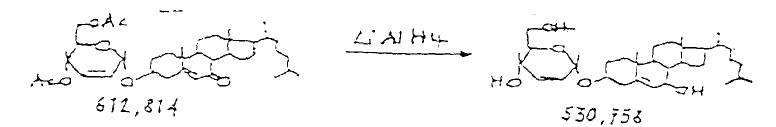

6.13 g (=. 0.01 mole) of pure compound from Example 2 with mp 113-115 °C were dissolved by heating in 100 ml peroxide-free ether which has been dried with metallic sodium and cooled to room temperature. A solution of 0.8-1.0 g (= 0.021 mole) LiAlH₄ in 100 ml absolute ether was added to a 500 ml three-necked flask with magnetic stirrer, reflux condensor and thermometer. The ethereal solution of the unsaturated aceto-7-keto-hex-2-ene-pyranoside was then added dropwise with sufficient stirring such that the reaction temperature did not substantially exceed 20 °C, if possible. After addition had been terminated, which may take up to two hours, stirring was continued for another 2 hours.

Afterwards, the reaction mixture was cooled in ice-water and treated drop by drop with H₂O until all H₂ (conducted to the outlet of the hood by means of a tube) had evolved.
H₂O-consumption was about 5.0 ml. On a larger scale, the use of CH₃COOC₂H₅ is recommended. In order to dissolve the LiAlO₂ formed, the solution was stirred with 16 ml of 10% H₂SO₄ and, after transfer to a 500 ml separating funnel, diluted with 100 ml of ether and shaken thoroughly. Thereby, the reaction product, which has separated as crystals, goes completely into solution. The acidic aqueous solution was extracted once with ether and the combined ethereal solutions washed with 100 mi of a saturated NaCl-solution in two portions of 50 ml each. After drying over anhydrous Na₂SO₄, the filtrate was kept in the refrigerator at -1 °C for 9 hours. The crystals thus obtained are collected by suction over a G4-suction filter and weighed.
Crude yield: 5.10 g (= 96.23% of theory)
mp: 165 -167 °C

This compound was dissolved in 25 ml of dioxane by heating and chromatographed on a column of silica gel (diameter 5.0 cm; height 70 cm) charged with 300 g of silica gel 40 (Merck Article 10180) using a solvent mixture consisting of dichloromethane 1 : acetone 1.

### Yield:

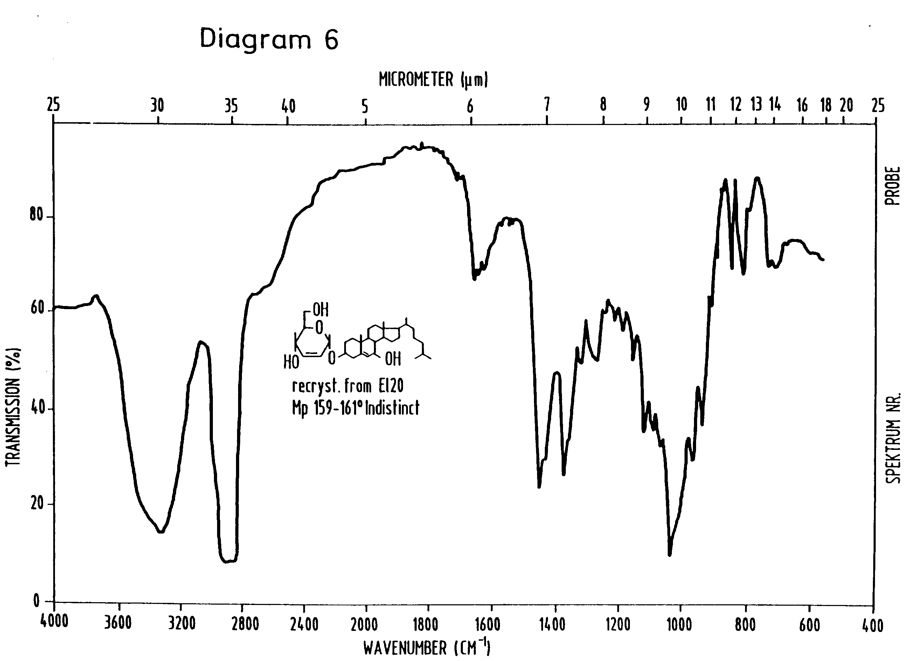

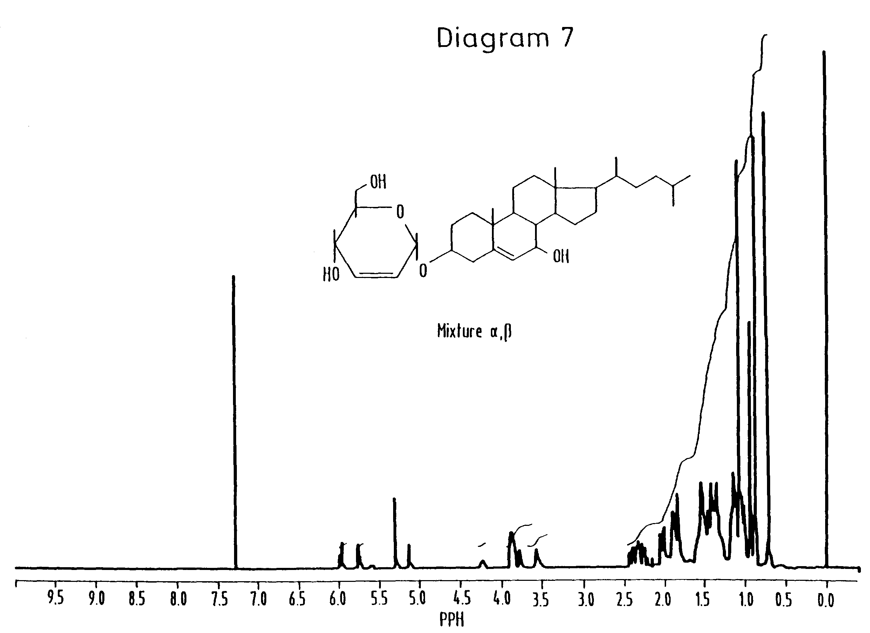

Fraction 1: 0.35 g (= 6.8%) 7α-OH-compound, mp: 161-195 °C.
Fraction 2: 4.60 g (= 90.2%) 7β-OH-compound, mp: 181-183 °C.
IR-spectrum: Diagram 6
NMR-spectrum: Diagram 7

## Claims

1. Method for the production of hex-2-ene pyranosides of alcohols and phenols, comprising glycosylating at least one member selected from the group consisting of an alcohol and a phenol by reaction with a glucal derivative in the presence of a catalytic amount of molecular iodine.

2. Method according to Claim 1, wherein said at least one member selected from the group consisting of an alcohol and a phenol is a hydroxy-steroid.

3. Method according to claim 2, wherein the hydroxy-steroid is Δ⁵-cholesten-3β-ol and the glucal derivative is 3,4,6-tri-O-acetyl-D-glucal.

4. Method according to claim 3, wherein the product formed by the glycosylation is 3-β-O-(4,6-O-diacetyl-2,3-dideoxy-D-erythro-α-hex-2-ene-pyranosyl)-Δ⁵-cholestene.

5. Method according to claim 4, further comprising oxidizing the 3-β-0-(4,6-O-diacetyl-2,3-dideoxy-D-erythro-α-hex-2-ene-pyranosyl)-Δ⁵-cholestene with an oxidizing agent to form a first product and then reducing said first product with a metal hydride reducing agent to form a second product.

6. Method according to claim 5, wherein said first product is 3-β-O-(4,6-O-diacetyl-2,3-dideoxy-D-erythro-α-hex-2-ene-pyranosyl)-Δ⁵-cholesten-7-one.

7. Method according to claim 5, wherein said second product is 3-β-O-(4,6-dihydroxy-2,3-dideoxy-D-erythro-α-hex-2-ene-pyranosyl)-Δ⁵-cholesten-7β-ol in admixture with 3-β-O-(4,6-dihydroxy-2,3-dideoxy-D-erythro-α-hex-2-ene-pyranosyl)-Δ⁵-cholesten-7α-ol.

8. Method according to claim 5, wherein said oxidizing agent is t-butyl-chromate, pyridine-chromium trioxide or pyridine chlorochromate.

9. Method according to claim 5, wherein said reducing agent is LiAlH₄ LiBH₄, NaBH₄ or KBH₄.

10. Method according to claim 7, further comprising separating the 3-β-O-(4,6-dihydroxy-2,3-dideoxy-D-erythro-α-hex-2-ene-pyranosyl)-Δ⁵-cholesten-7β-ol from the 3-β-O-(4,6-dihydroxy-2,3-dideoxy-D-erythro-α-hex-2-ene-pyranosyl)-Δ⁵-cholesten-7α-ol.

11. Method according to claim 10, wherein said separated 3-β-O-(4,6-dihydroxy-2,3-dideoxy-D-erythro-α-hex-2-ene-pyranosyl)-Δ⁵-cholesten-7β-ol is processed with a pharmaceutically acceptable carrier to form a pharmaceutical composition.

12. Compound of formula

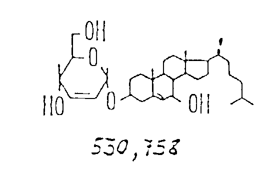

13. Medicament which comprises a pharmaceutically effective amount of 3-β-O-(4,6-dihydroxy-2,3-dideoxy-D-erythro-α-hex-2-ene-pyranosyl)-Δ⁵-cholesten-7β-ol together with another pharmaceutically acceptable ingredient.

14. Medicament according to Claim 13, wherein said another ingredient is selected from the group consisting of carriers, preservatives, solubilizers, stabilizers, humectants, emulsifiers, sweetening agents, dyes, scents, salts to modify the osmotic pressure, buffers, coating agents, antioxidants.

15. Medicament according to claim 13, wherein said amount is effective to treat symptoms of at least one member selected from the group consisting of cancer disease, geriatric disease, states of restlessness and states of weakness.

16. Use of a compound for the manufacture of a pharmaceutical for the treatment of at least one member selected from the group consisting of cancer disease, geriatric disease, states of restlessness and states of weakness, the compound having the formula: 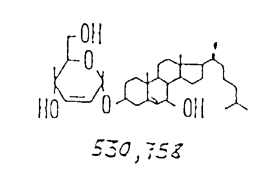 optionally in admixture with another pharmaceutically acceptable ingredient selected from the group consisting of carriers, preservatives, solubilizers, stabilizers, humectants, emulsifiers, sweetening agents, dyes, scents, salts to modify the osmotic pressure, buffers, coating agents, antioxidants.

17. Use according to claim 16, wherein said compound is formulated in a form selected from the group consisting of tablets, dragees, pills, gelatin capsules, soft capsules, suppositories, solutions, emulsions, suspensions, injectable solutions, troches, liniments, salves, ointments, creams.

18. Hex-2-ene pyranosides of alcohols and phenols formed by the process comprising glycosylating Δ⁵-cholesten-3β-ol by reaction with 3,4,6-tri-O-acetyl-D-glucal in the presence of a catalytic amount of molecular iodine to form a first product.

19. A method according to claim 1, wherein said member is an hydroxy-steroid and wherein said hydroxy-steroid is selected from the group containing cholic acid and derivatives, 25-hydroxy-cholesterol, 25-hydroxy-calciferol, Pregnenolone, 17α-hydroxy-pregnenolone, 17α-hydroxy-progesterone, 11-desoxy-corticosteron, 11-desoxy-cortisol, corticosterone, cortisol, cortisone, androsterone, testosterone, estrone, 17β-estradiol, estratriol-3, 16α, 17β, 3α, 5β-tetrahydro-corticosterone, urocortisol and allocortolone.

## Patentansprüche

1. Verfahren zur Herstellung von Hex-2-en-pyranosiden von Alkoholen und Phenolen, das die Glykosidierung von mindestens einem Mitglied, das aus der Gruppe ausgewählt wird, die aus einem Alkohol und einem Phenol besteht, durch Reaktion mit einem Glucalderivat in der Gegenwart einer katalytischen Menge molekularen Iods umfaßt.

2. Verfahren gemäß Anspruch 1, wobei zumindest ein Mitglied, das aus der Gruppe ausgewählt wird, die aus einem Alkohol und einem Phenol besteht, ein Hydroxysteroid ist.

3. Verfahren gemäß Anspruch 2, wobei das Hydroxysteroid Δ⁵-cholesten-3β-ol und das Glucalderivat 3,4,6-Tri-O-acetyl-D-glucal ist.

4. Verfahren gemäß Anspruch 3, wobei das durch die Glykosidierung gebildete Produkt 3-β-O-(4,6-O-diacetyl-2,3-dideoxy-D-erythro-α-hex-2-en-pyranosyl)-Δ⁵-cholesten ist.

5. Verfahren gemäß Anspruch 4, das weiterhin die Oxidation des 3-β-O-(4,6-O-diacetyl-2,3-dideoxy-D-erythro-α-hex-2-en-pyranosyl)-Δ⁵-cholestens mit einem Oxidationsmittel zur Bildung eines ersten Produkts und anschließend die Reduktion des ersten Produkts mit einem Metallhydrid-Reduktionsmittel zur Bildung eines zweiten Produkts umfaßt.

6. Verfahren gemäß Anspruch 5, wobei das erste Produkt 3-β-O-(4,6-O-diacetyl-2,3-dideoxy-D-erythro-α-hex-2-en-pyranosyl)-Δ⁵-cholesten-7-on ist.

7. Verfahren gemäß Anspruch 5, wobei das zweite Produkt 3-β-O-(4,6-dihydroxy-2,3-dideoxy-D-erythro-α-hex-2-en-pyranosyl)-Δ⁵-cholesten-7β-ol mit Beimischung von 3-β-O-(4,6-dihydroxy-2,3-dideoxy-D-erythro-α-hex-2-en-pyranosyl)-Δ⁵-cholesten-7α-ol ist.

8. Verfahren gemäß Anspruch 5, wobei das Oxidationsmittel t-Butylchromat, Pyridinchromtrioxid oder Pyridinchlorochromat ist.

9. Verfahren gemäß Anspruch 5, wobei das Reduktionsmittel LiAlH₄, LiBH₄, NaBH₄ oder KBH₄ ist.

10. Verfahren gemäß Anspruch 7, das ferner die Abtrennung des 3-β-O-(4,6-dihydroxy-2,3-dideoxy-D-erythro-α-hex-2-en-pyranosyl)-Δ⁵-cholesten-7β-ols vom 3-β-O-(4,6-dihydroxy-2,3-dideoxy-D-erythro-α-hex-2-en-pyranosyl)-Δ⁵-cholesten-7α-ol umfaßt.

11. Verfahren gemäß Anspruch 10, wobei das abgetrennte 3-β-O-(4,6-dihydroxy-2,3-dideoxy-D-erythro-α-hex-2-en-pyranosyl)-Δ⁵-cholesten-7β-ol mit einem pharmazeutisch annehmbaren Träger zur Bildung einer pharmazeutischen Zusammensetzung verarbeitet wird.

12. Eine Verbindung der Formel

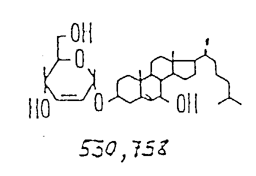

13. Arzneimittel, das eine pharmazeutisch wirksame Menge von 3-β-O-(4,6-dihydroxy-2,3-dideoxy-D-erythro-α-hex-2-en-pyranosyl)-Δ⁵-cholesten-7β-ol zusammen mit einem anderen pharmazeutisch annehmbaren Inhaltsstoff umfaßt.

14. Arzneimittel gemäß Anspruch 13, wobei der andere Inhaltsstoff aus der Gruppe ausgewählt wird, die aus Trägern, Konservierungsmitteln, Solubilisierungsmitteln, Stabilisierungsmitteln, Befeuchtungsmitteln, Emulgatoren, Süßmitteln, Farbstoffen, Riechstoffen, Salzen zur Veränderung des osmotischen Drucks, Puffern, Überzugsmitteln, Antioxidantien besteht.

15. Arzneimittel gemäß Anspruch 13, wobei die besagte Menge wirksam ist, um Symptome von zumindest einem Mitglied, das aus der Gruppe ausgewählt wird, die aus Krebserkrankung, geriatrischer Erkrankung, Unruhezuständen und Zuständen verminderten Antriebs besteht, zu behandeln.

16. Verwendung einer Verbindung für die Herstellung eines Pharmazeutikums für die Behandlung von zumindest einem Mitglied, das aus der Gruppe ausgewählt wird, die aus Krebserkrankung, geriatrischer Erkrankung, Unruhezuständen und Zuständen verminderten Antriebs besteht, wobei die Verbindung die Formel hat: 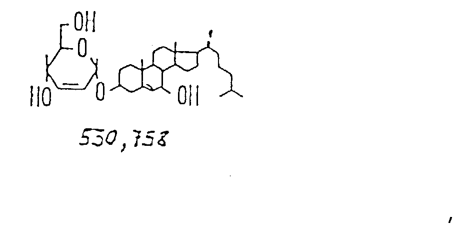 gegebenenfalls mit Beimischung eines anderen pharmazeutisch annehmbaren Inhaltsstoffes, der aus der Gruppe ausgewählt werden, die aus Trägern, Konservierungsmitteln, Solubilisierungsmitteln, Stabilisierungsmitteln, Befeuchtungsmitteln, Emulgatoren, Süßmitteln, Farbstoffen, Riechstoffen, Salzen zur Veränderung des osmotischen Drucks, Puffern, Überzugsmitteln, Antioxidantien besteht.

17. Verwendung gemäß Anspruch 16, wobei die Verbindung in einer Form formuliert ist, die aus der Gruppe ausgewählt wird, die aus Tabletten, Dragees, Pillen, Gelatinekapseln, Weichkapseln, Zäpfchen, Lösungen, Emulsionen, Suspensionen, Injektionslösungen, Pastillen, Einreibemitteln, Salben, medizinischen Salben, Cremes besteht.

18. Hex-2-en-pyranoside von Alkoholen und Phenolen, die durch das Verfahren gebildet werden, das die Glykosidierung von Δ⁵-cholesten-3β-ol durch Reaktion mit 3,4,6-Tri-O-acetyl-D-glucal in der Gegenwart einer katalytischen Menge molekularen Iods zur Bildung eines ersten Produkts umfaßt.

19. Verfahren gemäß Anspruch 1, wobei das Mitglied ein Hydroxysteroid ist, und wobei dieses Hydroxysteroid aus der Gruppe ausgewählt wird, die Cholsäure und Derivate, 25-Hydroxycholesterol, 25-Hydroxycalciferol, Pregnenolon, 17α-Hydroxypregnenolon, 17α-Hydroxyprogesteron, 11-Desoxycorticosteron, 11-Desoxycortisol, Corticosterone, Cortisol, Cortison, Androsteron, Testosteron, Estron, 17β-Estradiol, Estratriol-3, 16α, 17β, 3α, 5β-Tetrahydrocorticosteron, Urocortisol und Allocortolon enthält.

## Revendications

1. Procédé de préparation de hex-2-ène pyranosides d'alcools et de phénols, comprenant la glycosylation d'au moins un élément choisi dans le groupe consistant en un alcool et un phénol par réaction avec un dérivé de glucal en présence d'une quantité catalytique d'iode moléculaire.

2. Le procédé selon la revendication 1, dans lequel ledit au moins un élément choisi dans le groupe consistant en un alcool et un phénol est un hydroxy-stéroïde.

3. Le procédé selon la revendication 2, dans lequel l'hydroxy-stéroïde est le Δ⁵-cholestèn-3β-ol et le dérivé de glucal est le 3,4,6-tri-O-acétyl-D-glucal.

4. Le procédé selon la revendication 3, dans lequel le produit formé par la glycosylation est le 3-β-O-(4,6-O-diacétyl-2,3-didéoxy-D-érythro-α-hex-2-ène-pyranosyl)-Δ⁵-cholestène.

5. Le procédé selon la revendication 4, comprenant en outre l'oxydation du 3-β-O-(4,6-O-diacétyl-2,3-didéoxy-D-érythro-α-hex-2-ène-pyranosyl)-Δ⁵-cholestène par un agent oxydant pour former un premier produit et ensuite la réduction dudit premier produit par un hydrure métallique en tant qu'agent réducteur pour former un second produit.

6. Le procédé selon la revendication 5, dans lequel le premier produit est la 3-β-O-(4,6-O-diacétyl-2,3-didéoxy-D-érythro-α-hex-2-ène-pyranosyl)-Δ⁵-cholestèn-7-one.

7. Le procédé selon la revendication 5, dans lequel ledit second produit est le 3-β-O-(4,6-dihydroxy-2,3-didéoxy-D-érythro-α-hex-2-ène-pyranosyl)-Δ⁵-cholestèn-7β-ol en mélange avec le 3-β-O-(4,6-dihydroxy-2,3-didéoxy-D-érythro-α-hex-2-ène-pyranosyl)-Δ⁵-cholestène-7α-ol.

8. Le procédé selon la revendication 5, dans lequel ledit agent oxydant est le chromate de t-butyle, la pyridine-trioxyde de chrome ou le chlorochromate de pyridine.

9. Le procédé selon la revendication 5, dans lequel ledit agent réducteur est LiAlH₄, LiBH₄, NaBH₄ ou KBH₄.

10. Le procédé selon la revendication 7, comprenant en outre la séparation du 3-β-O-(4,6-dihydroxy-2,3-didéoxy-D-érythro-α-hex-2-ène-pyranosyl)-Δ⁵-cholestèn-7β-ol du 3-β-O-(4,6-O-dihydroxy-2,3-didéoxy-D-érythro-α-hex-2-ène-pyranosyl)-Δ⁵-cholestèn-7α-ol.

11. Le procédé selon la revendication 10, dans lequel ledit 3-β-O-(4,6-dihydroxy-2,3-didéoxy-D-érythro-α-hex-2-ène-pyranosyl)-Δ⁵-cholestèn-7β-ol isolé est traité avec un support pharmaceutiquement acceptable pour donner une composition pharmaceutique.

12. Le composé de formule:

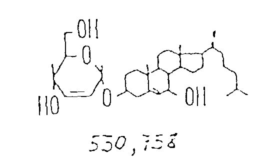

13. Un médicament qui comprend une quantité pharmaceutiquement efficace de 3-β-O-(4,6-dihydroxy-2,3-didéoxy-D-érythro-α-hex-2-ène-pyranosyl)-Δ⁵-cholestène-7β-ol en association avec un autre ingrédient pharmaceutiquement acceptable.

14. Un médicament selon la revendication 13, dans lequel ledit autre ingrédient est choisi dans le groupe consistant en supports, conservateurs, solubilisants, stabilisants, humidifiants, émulsifiants, agents édulcorants, colorants, parfums, sels modifiant la pression osmotique, tampons, agents d'enrobage et antioxydants.

15. Un médicament selon la revendication 13, dans lequel ladite quantité efficace pour traiter les symptômes d'au moins un état choisi dans le groupe consistant en maladie cancéreuse, maladie gériatrique, état d'agitation et état de faiblesse.

16. L'utilisation d'un composé pour la fabrication d'un produit pharmaceutique pour le traitement d'au moins un état choisi dans le groupe consistant en maladie cancéreuse, maladie gériatrique, état d'agitation et état de faiblesse, le composé répondant à la formule : 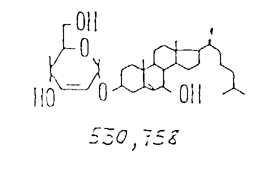 éventuellement en mélange avec d'autres ingrédients pharmaceutiquement acceptables choisis dans le groupe consistant en supports, conservateurs, solubilisants, stabilisants, humidifiants, émulsifiants, agents édulcorants, agents odorants, sels pour modifier la pression osmotique, tampons, agents d'enrobage et anti-oxydants.

17. Utilisation selon la revendication 16, dans laquelle ledit composé est formulé sous une forme choisie dans le groupe consistant en comprimés, dragées, pilules, capsules de gélatine, capsules molles, suppositoires, solution, émulsions, suspensions, solutions injectables, pastilles, liniments, baumes, pommades, crèmes.

18. Hex-2-ène pyranosides d'alcools et de phénoles obtenus par un procédé comprenant la glycosylation du Δ⁵-cholestène-3-β-ol par réaction avec le 3,4,6-triO-acétyl-D-glucal en présence d'une quantité catalytique d'iode moléculaire pour obtenir un premier produit.

19. Un procédé selon la revendication 1, dans lequel ledit élément est un hydroxy-stéroïde et dans lequel ledit hydroxy-stéroïde est choisi dans le groupe contenant l'acide cholique et ses dérivés, le 25-hydroxy-cholestérol, le 25-hydroxycalciférol, la pregnénolone, la 17α-hydroxy-pregnénolone, la 17α-hydroxyprogestérone, la 11-désoxy-corticostérone, le 11-désoxy-cortisol, la corticostérone, le cortisol, la cortisone, l'androstérone, la testostérone, l'estrone, le 17β-estradiol, l'estradiol-3, 16α, 17β, 3α, 5β-tétrahydro-corticostérone, l'urocortisol et l'allocortolone.
